# FASCICULE DE BREVET EUROPEEN

(11) **EP 1 938 828 B1**
(45) Date de publication et mention de la délivrance du brevet: **09.03.2016**
(21) Numéro de dépôt: 07291547.3
(22) Date de dépôt: 18.12.2007
(51) Int. Cl.: A61K 36/185, A61K 31/122, A61P 35/00

(54) **Utilisation de lupulones pour la prévention et la thérapie du cancer colorectal**
Anwendung von Lupulonen zur Vorbeugung und Therapie von Kolorektalkarzinomen
Use of lupulones for the prevention and treatment of colorectal cancer

(30) Priorité: 22.12.2006 FR 0611281
(43) Date de publication de la demande: 02.07.2008
(73) Titulaire: BRASSERIES KRONENBOURG, 67200 Strasbourg (FR)
(72) Inventeur: Raul, Francis, 67300 Schiltigheim (FR); Lobstein, Annelise, 67640 Lipsheim (FR); Gosse, Francine, 67000 Strasbourg (FR)
(74) Mandataire: Gevers & Orès

(56) Documents cités:
- WO-A-97/31630
- WO-A-03/000185
- WO-A-2006/053249
- US-A1- 2002 031 594
- NOZAWA HAJIME ET AL: "Cancer chemopreventive effects of beer hop (Humulus lupulus L.) on experimental rat colonic carcinogenesis and its physiological functions in vitro." CANCER EPIDEMIOLOGY BIOMARKERS & PREVENTION, vol. 12, no. 11 Part 2, novembre 2003 (2003-11), page 1317s, XP009087322 & SECOND ANNUAL AACR INTERNATIONAL CONFERENCE ON FRONTIERS IN CANCER PREVENTION RESEARCH : GENETICS, R; PHOENIX, ARIZONA, USA; OCTOBER 26-30, 2003 ISSN: 1055-9965
- CHEN WEI-JEN ET AL: "Mechanisms of cancer chemoprevention by hop bitter acids (beer aroma) through induction of apoptosis mediated by Fas and caspase cascades." JOURNAL OF AGRICULTURAL AND FOOD CHEMISTRY, vol. 52, no. 1, 1 janvier 2004 (2004-01-01), pages 55-64, XP002444086 ISSN: 0021-8561

## Description

La présente invention concerne le domaine de la prévention du cancer, et en particulier des cancers colorectaux.

Les cancers du côlon et du rectum sont la seconde cause de décès par cancer dans les pays développés, avec 945 000 cas/an et 452 000 décès/an dans le monde (OMS 2003). En France cela représente environ 36 000 cas/an avec un taux de survie sur 5 ans de 40-50%, soit 16 000 décès par an. Le risque de cancer colorectal est comparable chez les hommes et chez les femmes et, à 70 ans, environ 1 personne sur 25 aura été atteinte.

Aujourd'hui, la stratégie thérapeutique est basée sur la chirurgie et une chimiothérapie palliative à base de 5-FU modulé par l'acide folinique en association avec l'irinotécan ou le méthotrexate. Récemment, l'association 5-FU/oxaliplatine a reçu l'agrément de la FDA aux Etats-Unis pour le traitement en première ligne du cancer colorectal métastatique, malgré une neurotoxicité avérée. Le mauvais pronostique du cancer colorectal est lié à un diagnostic posé tardivement ce qui est d'autant plus regrettable que le développement de ce cancer s'étend sur plusieurs années, voire dizaines d'années. Ce développement est initié par l'apparition de lésions précancéreuses, qui sont d'abord des foyers de cryptes hyperprolifératives, ou foyers de cryptes anormales (FCA), au niveau de la muqueuse colique. Certains de ces FCA se transforment progressivement en polypes, lesquels peuvent éventuellement dégénérer ensuite en tumeurs. Il est clair qu'un dépistage précoce, au niveau des lésions précancéreuses, et leur traitement, permettrait une intervention bien plus efficace. Cette longue période de « transition » des lésions précancéreuses vers des tumeurs invasives offre en effet une large fenêtre d'intervention pour toute action pharmacologique visant à prévenir ou à freiner cette progression. De fait, le cancer colorectal paraît particulièrement bien indiqué pour le développement de stratégies de chimioprévention. A l'heure actuelle, les premiers essais (vitamine D + Calcium, aspirine, celecoxib etc...) ont d'ores et déjà été conduits avec des résultats décevants pour certains (vitamine D + Calcium) et plus encourageants pour d'autres (celecoxib, aspirine), mais ces résultats doivent encore être confirmés et affinés. D'autres essais sont en cours et cette voie de recherche dédiée à la chimioprévention du cancer colorectal est aujourd'hui en pleine expansion.

Dans la recherche de nouveaux composés utilisables en chimioprévention, certains constituants végétaux offrent des pistes intéressantes. Des études expérimentales montrent en effet que certains microconstituants présents dans l'alimentation, en particulier ceux apportés par les végétaux, pourraient avoir une action préventive sur le processus de la cancérogenèse (Wattenberg L.W., 1992, Inhibition of carcinogenesis by minor dietary constituents. Cancer Res. 52, 7suppl. 2085-2091). Selon les composés testés, un effet protecteur peut être observé soit durant la phase d'initiation, soit durant la phase de promotion et de progression du développement tumoral. Les mécanismes d'action possibles des phytoconstituants sont multiples. Ils peuvent agir sur des cibles génomiques, en modulant l'expression de gènes spécifiques, par exemple en agissant sur la méthylation de l'ADN, sur l'acétylation des histones. Ils peuvent également interagir directement sur certaines protéines régulatrices, des enzymes ou d'autres substances endo- ou exogènes (Wattenberg L.W., *supra* ; Wargovich M.J. et al., 2000, Efficacy of potential chemopreventive agents on rat colon aberrant crypt formation and progression. Carcinogenesis 21, 6: 1149-55). Ces phytoconstituants peuvent avoir des répercussions sur de nombreuses voies métaboliques, sur le statut rédox, sur la transduction de signaux intervenant dans le contrôle de la prolifération et de la mort cellulaire (Wattenberg L.W., *supra*).

Dans ce contexte, les inventeurs ont étudié les propriétés de différents constituants isolés d'extraits issus de la filière brassicole. Des études préliminaires sur des cellules en culture ont permis de mettre en évidence une activité antiproliférative importante de deux constituants des extraits préparés à partir de cônes de houblon (résines α et β).

Les résines β ou lupulones correspondent à des acides phénoliques alicycliques, différant des résines α ou humulones par la présence d'une chaîne isoprényle à la place d'un fonction hydroxyle acide. Ces résines, appelées aussi acides α et β, sont synthétisées exclusivement par les cônes de houblon (*Humulus lupulus L., Cannabaceae*). Elles existent naturellement sous 5 formes différentes, selon la longueur de la chaîne latérale (co : iso-butyroyle ; n : iso-valéroyle ; ad : méthyl 2-butyroyle ; pré : isopentoyle ; post : propionoyle), les formes co- et n- étant majoritaires.

Des extraits de houblon préparés par CO₂ supercritique concentrent ces résines α et β, très lipophiles. Ces extraits concentrés sont utilisés par l'industrie brassicole, qui les ajoute au cours de la cuisson du moût pour apporter arôme et amertume à la bière. Les lupulones, contrairement aux humulones, ne se retrouvent pas dans la bière mais sont piégées lors de la centrifugation du moût dans le "trouble whirlpool", qui est une matière de consistance boueuse contenant des protéines coagulées et des résidus de houblon et constitue actuellement un déchet industriel.

Les résultats expérimentaux présentés ci-dessous, obtenus sur un modèle animal de cancer du côlon, montrent le potentiel anticancérogène des lupulones, qui inhibent la phase d'initiation du cancer (formation de foyers de cryptes anormales, ou FCA), mais également les phases ultérieures (la transformation des FCA en polypes, et la transition des polypes en tumeurs).

La présente demande décrit donc en premier lieu sur l'utilisation de lupulones, pour la préparation d'une composition destinée à inhiber la formation de foyers de cryptes anormales au niveau de la muqueuse du côlon d'un mammifère. Selon cet aspect de la demande, il s'agit donc d'inhiber la phase initiale du processus qui conduit à la formation de tumeurs du côlon. Cette prévention des phases très précoces peut être particulièrement avantageuse chez des sujets qui présentent des facteurs de risque de cancer du côlon, notamment chez des sujets jeunes, en particulier ceux ayant des antécédents familiaux de cancer du côlon ou une prédisposition génétique telle que la polypose adénomateuse familiale ou le syndrome de Lynch.

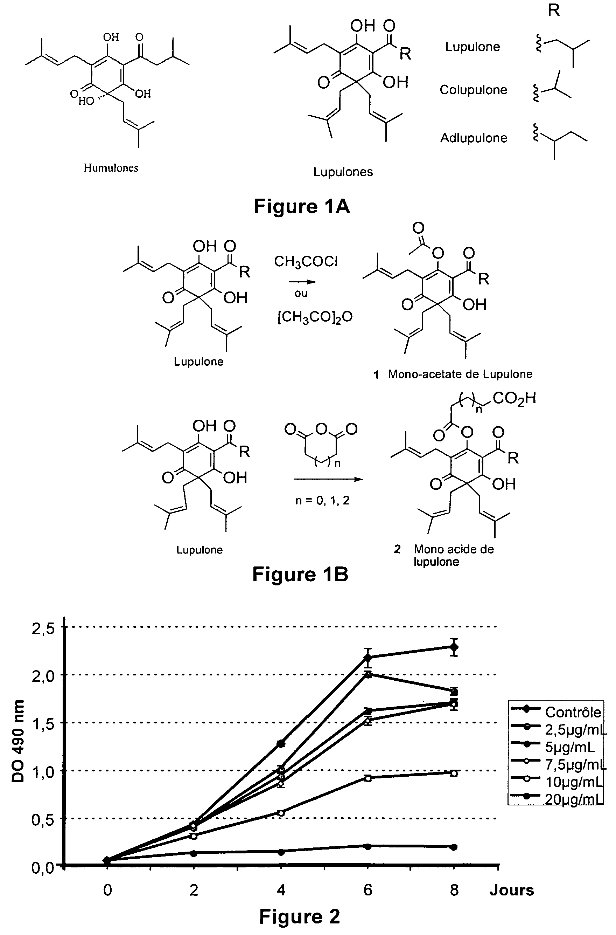

Dans le cadre de la présente invention, on entend par "lupulones" aussi bien les lupulones naturelles extraites du houblon, que des lupulones produites par synthèse chimique ou par hémisynthèse. L'invention peut être mise en oeuvre avec une forme particulière de lupulone purifiée (en particulier l'une des trois formes majoritaires : n-lupulone, colupulone ou adlupulone), ou avec un mélange de plusieurs formes de lupulones. En outre, les lupulones étant très sensibles à l'oxydation et peu solubles dans l'eau, il peut être avantageux, pour mettre en oeuvre la présente invention, d'utiliser un dérivé actif des lupulones. Des tels dérivés peuvent être obtenus, par exemple, en ajoutant des substituants au niveau des fonctions pseudo-phénoliques des différentes formes lupulones, sans toucher à l'intégrité de la structure de la lupulone, afin d'obtenir des composés prodrogues qui pourraient être retransformés en lupulone par des enzymes du type estérases ou hydrolases présentes dans le tube digestif. Dans la suite de ce texte, le terme "lupulones" désignera également de tels dérivés actifs des lupulones. A titre d'exemples de tels dérivés, on peut citer le monoacétate et des mono acides obtenus avec l'acide succinique ou glutarique comme représenté sur les schémas présentés à la figure 1.

Selon un autre aspect de l'invention, les lupulones sont utilisées pour la préparation d'une composition destinée à inhiber la formation de polypes au niveau de la muqueuse du côlon d'un mammifère présentant des foyers de cryptes anormales au niveau de cette muqueuse. Outre les catégories de sujets mentionnées ci-dessus (ayant des antécédents familiaux et/ou une prédisposition génétique au cancer du côlon), les sujets présentant déjà des foyers de cryptes anormales peuvent très avantageusement bénéficier d'un traitement par une composition selon cet aspect de l'invention, ainsi que les patients présentant un antécédent de polypectomie ou d'exérèse tumorale au niveau colique, ou les personnes âgées, notamment celles de plus de 65 ans ayant un mode de vie sédentaire et/ou une alimentation riche en viandes et pauvre en fruits et légumes.

La présente invention porte également sur l'utilisation de lupulones, pour la préparation d'une composition destinée à inhiber la cancérisation de polypes présents au niveau de la muqueuse du côlon d'un mammifère. Bien entendu, cette indication vise en particulier les personnes dont on sait ou on suspecte qu'elles ont des polypes coliques.

Ainsi, la présente invention décrit l'utilisation de lupulones pour préparer une composition destinée à prévenir les tumeurs colorectales, en particulier chez des individus présentant un ou plusieurs des facteurs de risque suivants :
- antécédents familiaux de cancer du côlon ;
- prédisposition génétique telle que la polypose adénomateuse familiale ou le syndrome de Lynch ;
- lésions précancéreuses au niveau du côlon ;
- antécédent de polypectomie ou d'exérèse tumorale au niveau colique ;
- âge supérieur à 65 ans combiné à un mode de vie sédentaire et/ou à une alimentation riche en viandes et pauvre en fruits et légumes.

Selon une mise en oeuvre particulière de l'invention, les lupulones sont utilisées sous forme d'extrait du "trouble whirlpool" obtenu dans le procédé de fabrication de la bière.

Selon un aspect particulier de l'invention, les lupulones sont utilisées dans une composition nutraceutique telle qu'un aliment fonctionnel ou un complément alimentaire. Par "aliment fonctionnel", on entend une préparation alimentaire à consommer comme un aliment normal bien qu'elle procure des bénéfices supérieurs à la nutrition classique. Un aliment fonctionnel comprenant des lupulones, ou enrichi en lupulones, fait également partie intégrante de la présnete invention. A titre d'exemples non limitatifs d'aliments fonctionnels selon l'invention, on peut citer les jus de fruits, sodas et autres boissons sans alcool, les produits à base de céréales, les yaourts et préparations lactées, les barres chocolatées, la margarine et autres matières grasses à tartiner, les biscuits, *etc.*

Des compléments alimentaires comportant des lupulones font également partie de l'utilisation selon la présente invention. Ces compléments peuvent se présenter, par exemple, sous forme de gélules, poudres ou comprimés. Bien entendu, ils peuvent contenir d'autres principes actifs, tels que de la vitamine D, du calcium, du magnésium, *etc.*

Alternativement, les lupulones peuvent être utilisées, dans le cadre de la présente invention, pour la préparation d'un médicament.

De façon avantageuse, les compositions utilisées dans le cadre de l'invention sont formulées de façon à permettre l'administration d'environ 30 mg/m² de lupulones par jour au mammifère à traiter. En particulier, lorsque ledit mammifère est un humain, la composition peut être formulée pour permettre l'administration de lupulones, en 1 à 3 prises quotidiennes, à raison de 10 à 100 mg/jour, de préférence 30 à 50 mg/jour.

Outre les dispositions qui précèdent, l'invention comprend encore d'autres dispositions, qui ressortiront des exemples expérimentaux ci-dessous, et des figures annexées.

### LEGENDE DES FIGURES

**Figure 1 : 1A** **:** formules développées des humulones et des lupulones. **1B :** schéma représentatif des réactions permettant d'obtenir du mono-acétate ou un mono-acide de lupulone.
**Figure 2** **:** croissance de cellules SW620 en culture, en présence de différentes concentrations de lupulones issues du trouble Whirlpool.
**Figure 3** **:** Phases de la cancérogenèse colique après induction par un cancérogène chimique (AOM) chez le rat (modèle de cancers sporadiques du colon). Les images ont été obtenues par coloration au bleu de méthylène et observation au microscope optique (X5).
**Figure 4** **:** Nombre total de cryptes ou de foyers observés après 6 semaines de traitement.
**Figure 5** **:** Nombre de foyers de cryptes hyperprolifératives/cm, après 7 mois de traitement.
**Figure 6** **:** **6A** **:** Nombre de tumeurs dans chacun des trois groupes d'animaux après 7 mois de traitement. **6B :** Répartition et volume des tumeurs dans les animaux de chaque groupe.

### EXEMPLES

### Exemple 1 : Protocole de récupération des résines 13 (lupulones) à partir du trouble whirlpool issu de la filière brassicole

Le "trouble whirlpool" est prélevé des usines de production (Société Brasseries Kronenbourg) et immédiatement congelé (-30°C) pour en faciliter le stockage.

Un aliquot est décongelé à température ambiante. 500 grammes sont prélevés et additionnés de 1 litre d'eau déminéralisée dans un récipient protégé de la lumière. Le mélange est agité à l'aide d'un agitateur magnétique à 400t/min durant 15 minutes, à température ambiante. Le mélange homogénéisé est réparti équitablement dans des godets de centrifugation légèrement opaques, de forte contenance (max 750 ml) et à couvercle vissable. Après équilibrage des poids des godets, ils sont centrifugés entre 200 et 300 g, pendant 10 min à 15°C. Les surnageants sont écartés. Cette opération est effectuée jusqu'à ce que les eaux de lavages soient parfaitement claires et limpides. Un minimum de trois lavages successifs à l'eau distillée est nécessaire.

Chaque culot de centrifugation obtenu est additionné de 250 ml d'éthanol pur, qualité CLHP (chromatographie liquide haute performance). A l'aide d'une spatule rigide en inox, le "trouble whirlpool" qui adhère au fond du godet est détaché. Une fois le couvercle du godet revissé, le godet est agité énergiquement pendant 1 à 2 min pour homogénéiser la solution. Les godets sont mis à centrifuger à 200 à 300 g pendant 15 min à 15°C. Cette opération est effectuée jusqu'à ce que les surnageants soient parfaitement incolores. Un minimum de trois extractions successives est nécessaire.

Les surnageants réunis constituent le mélange de lupulones, constitué majoritairement de co-lupulone et de n-lupulone. Ce mélange est filtré sous vide, à l'aide d'un verre fritté de porosité 2-3 µm, à l'abri de la lumière. Il est ensuite rapidement transvasé dans un ballon opaque puis concentré à sec, sous pression réduite, en ne dépassant pas 30°C. A la fin de l'évaporation, la solution prend l'aspect d'une suspension blanchâtre dans laquelle précipitent de petites boulettes marron foncé.

Cette solution concentrée est reprise dans un faible volume d'eau déminéralisée (40-50 ml) et à l'aide d'une spatule inox, l'extrait lipophile est décroché des parois du ballon. Le mélange est ensuite transvasé dans un tube à centrifuger, puis centrifugé entre 200 et 300 g pendant 10 min, à 15°C. Le surnageant est éliminé.

Un aliquot du culot est repris dans du méthanol et immédiatement analysé par CLHP (chromatographie liquide haute performance) pour vérifier l'absence de dégradation des lupulones. Si l'absence d'hulupones (produits d'oxydation des lupulones) est confirmée, le culot est ensuite conservé à -30°C.

Il sera décongelé à température ambiante et à l'abri de la lumière, puis immédiatement utilisé (préparation de solutions extemporanées).

Le rendement massique moyen est de 0,8 % soit 8g de lupulones récupérées à partir d'1 kg de "trouble whirlpool". Leur degré de pureté relatif (λ_{270 nm}) est d'au minimum 80%.

### Exemple 2 : Effets des lupulones sur la prolifération de cellules in vitro

L'effet des lupulones et d'autres composés issus du houblon a été testé dans un premier temps sur 4 lignées cellulaires humaines de cancer colique ou colorectal : HT29, CaCo-2, SW280 et SW620.

Les principaux constituants des extraits préparés à partir de cônes de houblon et recherchés par l'industrie brassicole pour leurs qualités aromatiques et amérisantes (résines α et β) présentent une activité antiproliférative supérieure aux autres constituants naturels étudiés. En particulier, les lupulones présentent d'excellents résultats. Le Tableau 1 ci-dessous montre les résultats obtenus sur les cellules CaCo-2.

**Tableau 1 : effet de différents extraits sur la croissance des cellules CaCo-2**

| | Echantillons | Teneurs moyennes dans la bière | P.M. | % pureté | IC₅₀ µM | IC₅₀ µg/ml |
|---|---|---|---|---|---|---|
| Résines α | humulones: α-acides ...% nH +...% coH | 0.5 mg / L | 355 | 74 | 22,5 | 8 |
| | humulones = α-acides 85% nH + 12% coH | | | 97 | 12,7 | 4,5 |
| | isohumulones: iso-α-acides isocoH + isoH + isoadH | 15 mg/ L | 538 | 64,5 | 28 | 15 |
| | tétrahydro-isohumulones: tétrahydro-isocoH + isoadH | 3mg/L | 360 | 97,9 | 11,1 | 4 |
| Résines β | lupulones = β acides *35% nL* + *25% coL* | -- | 407 | 60 | 11 | 4,5 |
| | | Whirlpool | | | | |
| Polyphénols | xanthohumol | 0.04 - 4 mg / L | 354 | > 95 | 10 | 3.5 |
| | naringénine | | 272 | > 95 | | 25 |
| | 8-prényl-naringénine | | 340 | 95 | 20,6 | 7 |

Une inhibition de croissance de 50% a en effet été observée avec les lupulones à la concentration de 11 µM. Les inventeurs ont en outre démontré que ces effets anti-prolifératifs sont dus à l'activation d'un processus de mort programmée dans ces cellules, induit par les lupulones (altération du cycle cellulaire, activation de caspases, fragmentation de l'ADN).

L'effet des hulupones, produit de dégradation des lupulones, est moins important, puisque pour ces composés, testés sur les cellules CaCo-2, IC₅₀ = 20µg/ml.

La figure 2 montre l'effet de différentes concentrations de lupulones issues du trouble Whirlpool sur la croissance de cellules SW620 en culture. Pour ces cellules, la concentration entraînant une inhibition de croissance de 50% est de 8,5 µg/ml.

### Exemple 3 : Effets des lupulones sur la formation de lésions précancéreuses

Les effets des lupulones ont ensuite été testés dans un modèle animal de cancérogenèse colorectale. Ce modèle reproduit en un an les différentes étapes de la cancérogenèse colorectale observée chez l'homme qui se déroulent sur une période de plusieurs dizaines d'années dans les cas de cancers colorectaux sporadiques qui représentent plus de 80% des cas.

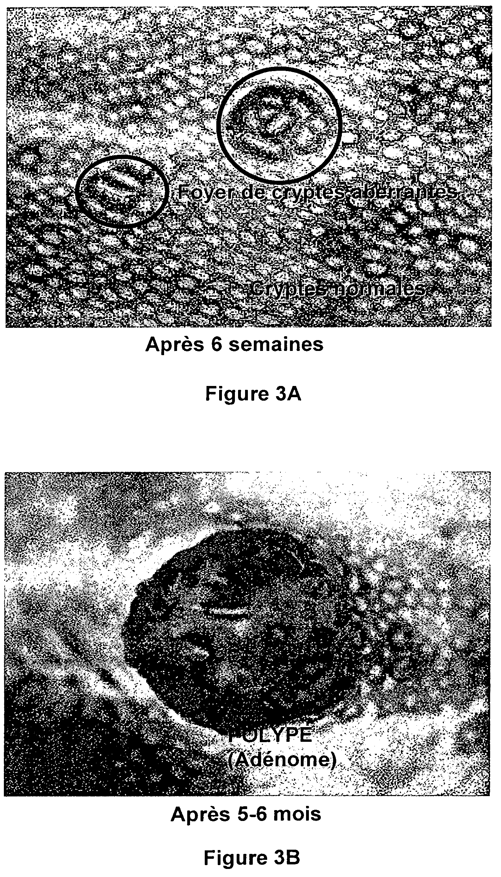

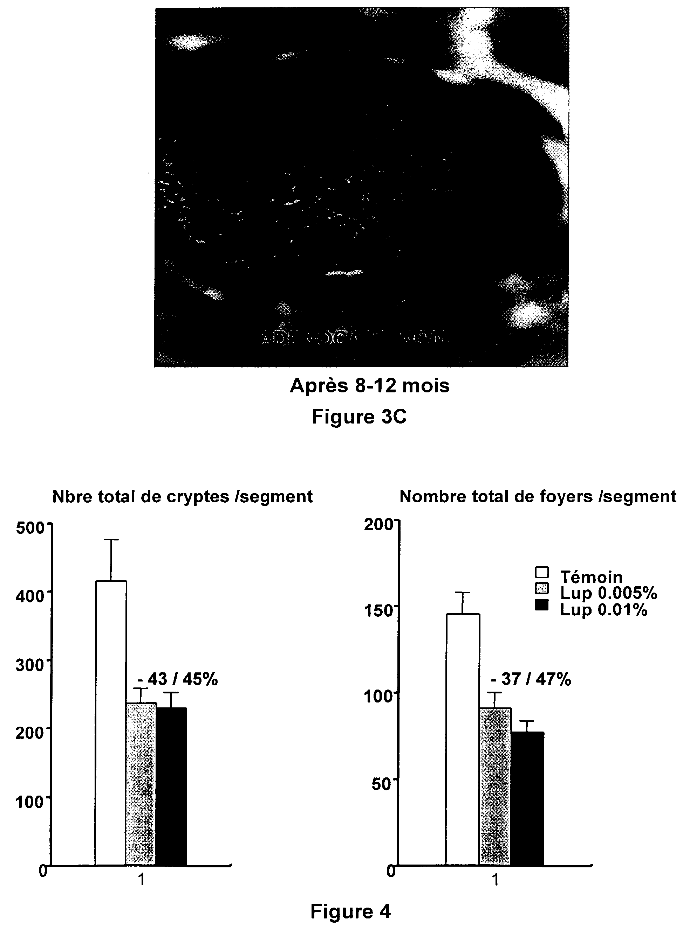

Des rats Wistar adultes d'un poids de 230-250g (n=12) ont reçu par voie intrapéritonéale une injection d'azoxyméthane (AOM : 15 mg/kg de poids corporel), une fois par semaine pendant 2 semaines, dans le but d'initier un processus de cancérogenèse colorectale. La figure 3 illustre les différentes phases de la cancérogénèse colique après induction par l'AOM chez le rat, en absence de traitement.

Une semaine après la dernière injection d'AOM, les animaux ont été répartis aléatoirement en 3 groupes de 6 animaux.

Les groupes expérimentaux ont reçu comme boisson une solution contenant 0.01% ou 0.005% (V/V) de l'extrait lupulone dans de l'éthanol à 1% (VN) et 0.001% d'acide ascorbique (V/V). Cette solution a été renouvelée tous les jours pendant la période expérimentale (6 semaines). Le groupe AOM-contrôle a reçu 1% d'éthanol et 0.001% d'acide ascorbique dans les mêmes conditions. L'éthanol favorise la dissolution des lupulones et l'acide ascorbique (Vitamine C) est un antioxydant puissant permettant d'éviter l'oxydation et la dégradation des lupulones.

La consommation moyenne quotidienne de lupulones, pour le groupe ayant reçu la solution de l'extrait lupulone la plus diluée, est de 5 mg/kg de poids corporel, soit 30 mg/m².

Six semaines après le début du traitement avec les lupulones, le côlon a été prélevé sous anesthésie. La mesure de cryptes anormales hyperprolifératives (précancéreuses) a été réalisée dans la moitié distale du côlon (longueur du segment: 5-6 cm). Le segment prélevé a été lavé dans une solution de NaCl 0.9%, ouvert longitudinalement puis épinglé à plat (face muqueuse sur le dessus) au fond d'une boîte de Pétri et immergé dans une solution Krebs Ringer contenant 4% de formaldéhyde. Pour l'analyse, le segment a été placé pendant 5-10 min dans une solution de bleu de méthylène à 0.5% pour coloration, puis rincé dans une solution de Krebs Ringé et placé sur une lame de verre pour observation. Le segment a été observé au microscope optique (grossissement 5X) et le nombre de foyers de cryptes aberrantes a été compté. Les critères retenus pour l'identification des cryptes aberrantes hyperprolifératives sont: (a) un épaississement du contour de la crypte, (b) sa dilatation, (c) la formation de foyers contenant plusieurs cryptes anormales (d) une rétention de la coloration liée à la présence d'amas cellulaires (figure 3A).

Les résultats illustrés dans la figure 4 démontrent que l'administration de lupulones induit une diminution de près de 50% du nombre de lésions précancéreuses dans la muqueuse du côlon.

### Exemple 4 : Effets des lupulones sur la formation de tumeurs colorectales

Dans une expérience complémentaire, les effets des lupulones ont été testés dans le même modèle de cancérogenèse colorectale, mais à un stade plus avancé du processus cancéreux. Comme décrit précédemment, une semaine après la dernière injection d'AOM, les animaux ont été répartis aléatoirement en 3 groupes de 6 animaux.

Les 3 groupes de rats ont été traités dans les mêmes conditions que précédemment, à ceci près que les teneurs en lupulones administrées aux deux groupes expérimentaux étaient plus faibles : 0.001% et 0.005% au lieu de 0.005% et 0.01% et que la solution d'éthanol utilisée pour dissoudre les lupulones (groupes expérimentaux) et administrée également au groupe contrôle, est une solution à 0,5% et non 1%. L'administration de lupulones a été réalisée comme précédemment et a été prolongée pendant une durée totale de 7 mois, période à laquelle les animaux traités par l'AOM développent des tumeurs.

Le suivi de l'évolution pondérale des animaux n'a montré aucune variation significative de poids entre le groupe contrôle et les groupes expérimentaux, et une absence totale de toxicité des lupulones administrées dans ces conditions.

Pour suivre l'évolution des polypes et leur transformation en adénocarcinome sans sacrifier les animaux, les inventeurs ont utilisé une technique originale de colonoscopie virtuelle par un scanner permettant, par un système d'acquisition anatomique, d'obtenir de très bonnes résolutions (jusqu'à 27 µm) tout en assurant une synchronisation sur le cycle respiratoire (Micro-CTscanner X, Imtek, San Diego, USA). Tout comme lors d'une colonoscopie chez l'homme, des produits de contraste qui permettent de différencier les tissus sains des tissus pathologiques ont été utilisés. Cette technique permet de détecter la présence (ou l'absence) de polypes et de tumeurs dans le côlon chez un animal vivant et de suivre l'évolution tumorale dans le temps.

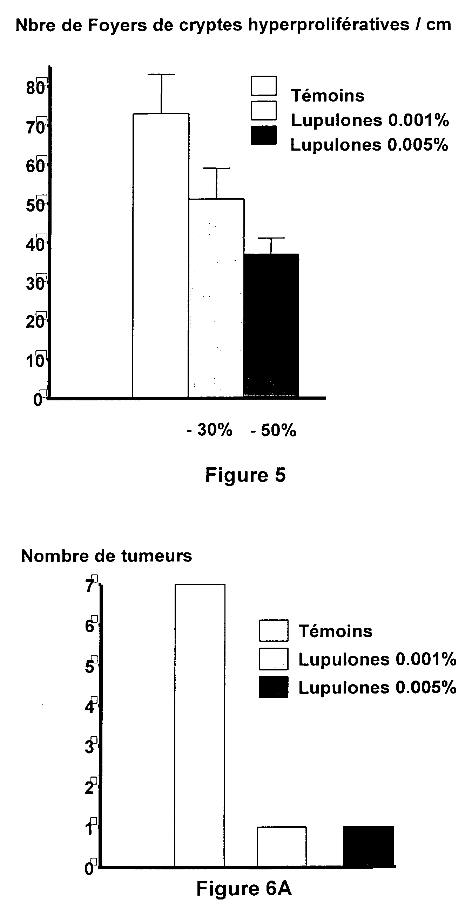

La figure 5 montre le nombre de foyers cryptiques aberrants présents par centimètre dans le segment terminal du côlon. Une réduction de 50 % du nombre de foyers est observée, ce qui confirme les résultats obtenus précédemment à un stade plus précoce.

Il apparaît en outre que la taille des foyers de cryptes hyperprolifératives est en moyenne plus basse chez les animaux traités par les lupulones que chez les animaux témoins, et qu'on observe 2 à 4 fois moins de foyers de grande taille (formés par plus de 10 cryptes) chez les animaux traités que chez les animaux témoins (Tableau 2).

**Tableau 2 : Dimension des foyers (nombre de cryptes/foyer)**

| | 1 crypte (%) | 2 cryptes (%) | 3 cryptes (%) | 4 cryptes (%) | 5 cryptes (%) | 6 cryptes (%) | ≥ 10 cryptes (nombre total) |
|---|---|---|---|---|---|---|---|
| Témoins | 8 ± 2 | 18 ± 5 | 16 ± 2 | 13 ± 1 | 6 ± 1 | 39 ± 7 | 12 ± 3 |
| 0.001% | 11 ± 1 | 21 ± 2 | 21 ± 2 | 13 ± 1 | 9 ± 1 | 24 ± 3 | 6 ± 1 |
| 0.005% | 12 ± 3 | 22 ± 2 | 17 ± 2 | 15 ± 2 | 10 ± 2 | 22 ± 6 | 3 ± 1 |

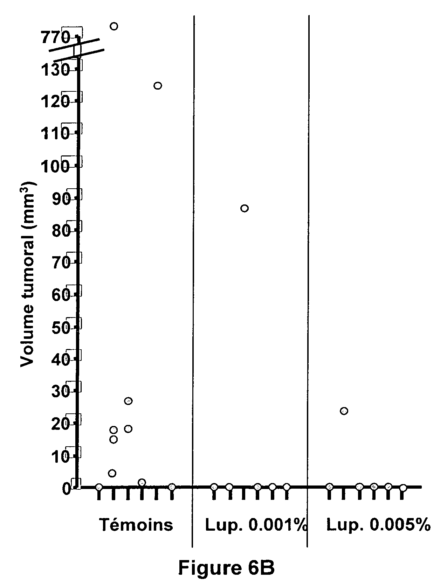

De plus, on observe après 7 mois un effet inhibiteur particulièrement important des lupulones sur la formation des tumeurs. Une analyse du nombre de tumeurs présentes dans la totalité du côlon montre qu'un total de 8 tumeurs est dénombré dans les côlons des rats AOM-contrôles alors qu'une seule tumeur est détectée dans chacun des groupes de rats traités avec les lupulones (figure 6A). Une analyse du nombre et de la taille des tumeurs dans le côlon de chaque animal est particulièrement révélatrice des propriétés anticancérogènes des lupulones sur la muqueuse colique. Des tumeurs sont ainsi présentes dans 4 rats sur 6 (67%), dont 2 tumeurs supérieurs à120 mm³ dans le groupe AOM-contrôle, alors qu'un seul rat sur 6 (20%) présente une tumeur dans chacun des groupes traités par les lupulones, la tumeur en question étant de taille moyenne (85 mm³) ou petite (25 mm³) (figure 6B).

Ces résultats montrent clairement que dans le modèle de rat utilisé ici, les lupulones inhibent la formation des polypes à la concentration de 4 mg/kg/j. Pour obtenir un dosage équivalent, chez l'homme, la consommation journalière devrait être de 30 à 50 mg (soit environ 30 mg/m²).

## Revendications

1. Lupulones, pour utilisation comme agent destiné à inhiber la formation de polypes au niveau de la muqueuse du côlon d'un mammifère présentant des foyers de cryptes anormales au niveau de cette muqueuse.

2. Lupulones, pour utilisation comme agent destiné à inhiber la cancérisation de polypes présents au niveau de la muqueuse du côlon d'un mammifère.

3. Lupulones pour une utilisation selon la revendication 1 ou la revendication 2, dans une composition destinée à être administrée à des individus présentant un ou plusieurs des facteurs de risque suivants :
- antécédents familiaux de cancer du côlon ;
- prédisposition génétique telle que la polypose adénomateuse familiale ou le syndrome de Lynch ;
- lésions précancéreuses au niveau du côlon ;
- antécédent de polypectomie ou d'exérèse tumorale au niveau colique ;
- âge supérieur à 65 ans combiné à un mode de vie sédentaire et/ou à une alimentation riche en viandes et pauvre en fruits et légumes.

4. Lupulones pour une utilisation selon l'une quelconque des revendications 1 à 3, **caractérisées en ce qu'**elles sont utilisées sous forme d'extrait du trouble whirlpool qui est obtenu dans le procédé de fabrication de la bière.

5. Lupulones pour une utilisation selon l'une quelconque des revendications 1 à 3, **caractérisées en ce qu'**elles sont produites par synthèse chimique ou hémisynthèse.

6. Lupulones pour une utilisation selon l'une quelconque des revendications 1 à 5, **caractérisées en ce qu'**elles sont utilisées dans un aliment fonctionnel.

7. Lupulones pour une utilisation selon l'une quelconque des revendications 1 à 5, **caractérisées en ce qu'**elles sont utilisées dans un complément alimentaire.

8. Lupulones pour une utilisation selon l'une quelconque des revendications 1 à 5, pour utilisation comme médicament.

9. Lupulones pour une utilisation selon l'une quelconque des revendications 1 à 8, pour utilisation dans une composition destinée à un humain.

10. Lupulones pour une utilisation selon la revendication 9, pour utilisation dans une composition formulée pour permettre l'administration, en 1 à 3 prises quotidiennes, de 10 à 100 mg /jour, de préférence 30 à 50 mg/jour.

## Patentansprüche

1. Lupolone zur Verwendung als Mittel, das zur Hemmung der Bildung von Polypen im Bereich der Schleimhaut des Darms eines Säugetiers, das anormale Cryptenherde im Bereich dieser Schleimhaut aufweist, bestimmt ist.

2. Lupolone zur Verwendung als Mittel, das zur Hemmung der Kanzerisierung von Polypen, die im Bereich der Schleimhaut des Darms eines Säugetiers vorhanden sind, bestimmt ist.

3. Lupolone für eine Verwendung nach Anspruch 1 oder Anspruch 2 in einer Zusammensetzung, die zur Verabreichung an Individuen bestimmt ist, die einen oder mehrere der folgenden Risikofaktoren aufweisen:
- Darmkrebsvorgeschichte in der Familie,
- genetische Prädisposition wie familiäre adenomatöse Polyposis oder Lynch-Syndrom,
- präkanzeröse Läsionen im Bereich des Darms,
- Vorgeschichte einer Polypektomie oder Tumorentfernung im Bereich des Darms,
- Alter über 65 Jahre, kombiniert mit einem sitzenden Lebensstil und/oder mit einer fleischreichen und obst- und gemüsearmen Ernährung.

4. Lupolone für eine Verwendung nach einem der Ansprüche 1 bis 3, **dadurch gekennzeichnet, dass** sie in Form von Whirlpooltrubextrakt verwendet werden, der bei der Bierherstellung gewonnen wird.

5. Lupolone für eine Verwendung nach einem der Ansprüche 1 bis 3, **dadurch gekennzeichnet, dass** sie durch chemische Synthese oder Hemisynthese gewonnen werden.

6. Lupolone für eine Verwendung nach einem der Ansprüche 1 bis 5, **dadurch gekennzeichnet, dass** sie in einer Funktionsnahrung verwendet werden.

7. Lupolone für eine Verwendung nach einem der Ansprüche 1 bis 5, **dadurch gekennzeichnet, dass** sie in einer Nahrungsergänzung verwendet werden.

8. Lupolone für eine Verwendung nach einem der Ansprüche 1 bis 5 für eine Verwendung als Arzneimittel.

9. Lupolone für eine Verwendung nach einem der Ansprüche 1 bis 8 für eine Verwendung in einer Zusammensetzung, die für einen Menschen bestimmt ist.

10. Lupolone für eine Verwendung nach Anspruch 9 für eine Verwendung in einer Zusammensetzung, die formuliert ist, um die Verabreichung in 1 bis 3 täglichen Gaben von 10 bis 100 mg/Tag, vorzugsweise 30 bis 50 mg/Tag, zu erlauben.

## Claims

1. Lupulones, for use as an agent intended to inhibit the formation of polyps at the mucosa of the colon of a mammal having abnormal crypt foci at this mucosa.

2. Lupulones, for a use of as an agent intended for inhibiting cancerization of polyps present at the mucosa of the colon of a mammal.

3. Lupulones for a use according to claim 1 or claim 2, in a composition intended to be administered to individuals having one or several of the following risk factors:
- colon cancer family history;
- genetic predisposition such as familial adenomatous polyposis or Lynch's syndrome;
- pre-cancer lesions at the colon;
- history of polypectomy or tumoral surgical removal at the colon;
- above 65 years old, combined with a sedentary lifestyle and/or with a diet rich in meat and low in fruits and vegetables.

4. Lupulones for a use according to any of claims 1 to 3, **characterized in that** they are used in the form of whirlpool extract which is obtained in the process for making beer.

5. Lupulones for a use according to any of claims 1 to 3, **characterized in that** they are produced by chemical synthesis or hemi-synthesis.

6. Lupulones for a use according to any of claims 1 to 5, **characterized in that** they are used in a functional food.

7. Lupulones for a use according to any of claims 1 to 5, **characterized in that** they are used in a food supplement.

8. Lupulones for a use according to any of claims 1 to 5, for use as a drug.

9. Lupulones for a use according to any of claims 1 to 8, for use in a composition intended for a human.

10. Lupulones for a use according to claim 9, for use in a composition formulated for allowing administration, 1 to 3 times per day, from 10 to 100 mg/day, preferably 30 to 50 mg/day.
